# EUROPÄISCHE PATENTSCHRIFT

(11) **EP 0 342 427 B1**
(45) Veröffentlichungstag und Bekanntmachung des Hinweises auf die Patenterteilung: **05.01.1994**
(21) Anmeldenummer: 89107876.8
(22) Anmeldetag: 29.04.1989
(51) Int. Cl.: C07D 493/10, A61K 31/335

(54) **Strobilurinderivate, ihre Herstellung und Verwendung**
Strobilurinderivatives, their preparation and use
Dérivés de strobilurine, leur préparation et utilisation

(30) Priorität: 06.05.1988 DE 3815484
(43) Veröffentlichungstag der Anmeldung: 23.11.1989
(73) Patentinhaber: BASF Aktiengesellschaft, 67063 Ludwigshafen (DE)
(72) Erfinder: Daum, Lothar, Dr., D-6701 Otterstadt (DE); Keilhauer, Gerhard, Dr., D-6701 Dannstadt-Schauernheim (DE); Lorenz, Gisela, Dr., D-6730 Neustadt (DE); Ammermann, Eberhard, Dr., D-6700 Ludwigshafen (DE); Anke, Timm, Prof. Dr., D-6750 Kaiserslautern (DE); Weber, Wolfgang, D-6750 Kaiserslautern (DE); Steglich, Wolfgang, Prof. Dr., D-5300 Bonn-Roettgen (DE); Steffan, Bert, Dr., D-5308 Rheinbach (DE); Scherer, Angela, D-5300 Bonn 1 (DE)

(56) Entgegenhaltungen:
- THE JOURNAL OF ANTIBIOTICS, Band 36, Nr. 6, Juni 1983, Seiten 661-666, Japan Antibiotics Research Association, Tokyo, JP; T. ANKE et al.: "Antibiotics from basidiomycetes. XVIII. Strobilurin C and oudemansin B, two new antifungal metabolites from Xerula species (Agaricales)"

## Beschreibung

Die vorliegende Erfindung betrifft neue Strobilurinderivate, Verfahren zu deren Herstellung sowie deren Verwendung bei der Bekämpfung von Krankheiten und bei der Bekämpfung von pflanzenpathogenen Fungi.

Im Journal of Antibiotics, 32 (1979) pp. 1112-1117, Bd. 36 (1983), pp. 661-666, und in "Cellular Regulation and Malignant Growth". Springer Verlag, Berlin 1985, pp. 169-176 sind die Verbindungen Strobilurin und Oudemansin und deren Derivate beschrieben. Diese Verbindungen Zeichnen sich durch eine starke antifungische und cytostatische Aktivität aus.

Die Strobilurine A, B und C sowie die Oudemansine A und B wurden aus den Gattungen Strobilurus, Oudemansiella, Xerula, Cyphellopsis, Hydropus und Mycena isoliert. Alle Verbindungen hemmen die Atmung von Eukaryonten und dadurch das Wachstum von Pilzen und Zellen. Die Strobilurine und Oudemansine binden reversibel an das bₜ-Zentrum von Cytochrom b in Komplex III der mitochondrialen Atmungskette.

Es wurde nun gefunden, daß Strobilurinderivate der Formel I 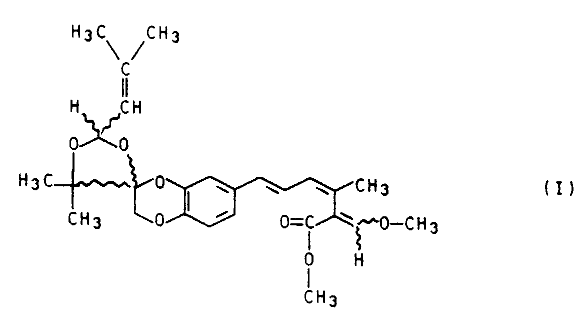
eine bessere Wirkung besitzen.

Die neuen Verbindungen lassen sich herstellen, indem man einen die Strobilurinderivate der Formel I bildenden Mikroorganismus der Gattung Crepidotus züchtet und die Strobilurinderivate der Formel I aus dem Mycel isoliert.

Mikroorganismen der Gattung Crepidotus sind von den bekannten Hinterlegungsstellen erhältlich. Hieraus lassen sich durch einen einfachen Handversuch diejenigen ermitteln, die die neuen Verbindungen bilden. Ein solcher Mikroorganismus ist der Stamm DSM 4545, der von der Deutschen Sammlung für Mikroorganismen in Braunschweig bezogen werden kann.

Zur Züchtung der Mikroorganismen eignen sich die üblichen Nährmedien, die Kohlenstoffquellen, Stickstoffquellen, anorganische Salze und gegebenenfalls geringe Mengen an Spurenelementen und Vitaminen enthalten. Als Stickstoffquellen können anorganische oder organische Stickstoffverbindungen oder Materialien, die diese Verbindungen enthalten, verwendet werden. Beispiele sind: Ammoniumsalze, Maisquellwasser, Bierhefeautolysat, Sojabohnenmehlhydrolysat, Weizengluten, Hefeextrakt, Hefe, Harnstoff und Kartoffelprotein. Besonders vorteilhaft ist die Verwendung von Maisquellwasser. Als Kohlenstoffquellen können Zucker, wie D-Glucose, Mannose oder Galaktose, Polyalkohole, wie Mannit, oder Alkohole, wie Ethanol, verwendet werden.

Beispiele für anorganische Salze sind die Salze von Calcium, Magnesium, Mangan, Kalium, Zink, Kupfer, Eisen und anderen Metallen. Als Anion der Salze ist besonders das Phosphation zu nennen. Gegebenenfalls werden dem Nährmedium Wachstumsfaktoren zugesetzt, wie z.B. Pantothensäure, p-Aminobenzoesäure und Thiamin. Das Mischungsverhältnis der genannten Nährstoffe hängt von der Art der Fermentation ab und wird im Einzelfall festgelegt.

Die erfindungsgemäßen Verbindungen werden nach der Fermentation aus dem Mycel isoliert. Hierzu wird das Mycel von der Kulturbrühe abgetrennt und getrocknet. Das Mycel wird mit einem polaren Lösungsmittel wie einem niedermolekularen Alkohol extrahiert, wobei die erfindungsgemäßen Verbindungen in Lösung gehen. Der so erhaltene Extrakt wird eingeengt und der Rückstand chromatographisch gereinigt.

Die neuen Verbindungen - insbesondere die Substanz des Beispiels 1 zeigen eine gute antivirale, antiproliferative und zytotoxische Aktivität. Darüber hinaus besitzen sie eine gute antifungische Wirkung.

Sie zeichnen sich weiter durch eine gute Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Sie lassen sich daher zur Behandlung von festen Tumoren, wie Carcinome im Mamma-, Lungen-, Colon- und Nierenbereich, von akuter und chronischer Leuxämie sowie von viralen Erkrankungen, wie Herpes-Infektionen einsetzen.

Besonders interessant sind die neuen Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Uncinula necator an Reben,
Puccinia-Arten an Getreide,
Rhizoctonia-Arten an Baumwolle und Rasen,
Venturia inaequalis (Schorf) an Äpfeln,
Helminthosporium-Arten an Getreide,
Septoria nodorum an Weizen,
Botrytis cinerea (Grauschimmel) an Erdbeeren, Reben.
Cercospora arachidicola an Erdnüssen,
Pseudocercosporella herpotrichoides an Weizen, Gerste,
Fusarium- und Verticillium-Arten an verschiedenen Pflanzen,
Plasmopara Viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

### Beispiel 1

200 ml eines Vorkulturmediums, das 20 g/l Glucose, 5 g/l Pepton, 5 g/l Hefeextrakt, 0,5 g/l KH₂PO₄, 1 g/l MgSO₄ , 7H₂O, 10 mg/l FeCl₃, 1,78 mg/l ZnSO₄ und 73,5 mg/l CaCl₂ enthält, wurden in einem 500 ml Erlenmeyerkolben mit seitlichem Einstich eingebracht. Das Medium wurde mit einem myceldurchwachsenen Agarstückchen einer Crepidotus-Kultur (DSM 4545) angeimpft und 14 Tage bei Raumtemperatur und 120 Upm inkubiert. Hierauf wurde der Inhalt in einen 25 l fassenden Fermenter überführt, der 20 l des gleichen Mediums enthielt. Der pH-Wert des Mediums betrug 5,8. Die Kultur wurde dann 21 Tage bei Raumtemperatur unter Rühren mit 200 U/min und mit einer Belüftung von 2 l/min inkubiert.

Anschließend wurden 20 l der Kulturbrühe abfiltriert und der erhaltene Mycelkuchen lyophilisiert. Das Lyophilisat (271 g) wurde mit 10 l Methanol extrahiert. Die methanolische Mycelsuspension wurde abfiltriert und unter vermindertem Druck bei 45°C eingedampft. Der erhaltene ölige Extrakt (98 g) wurde an Kieselgel adsorbiert. Dieses wurde auf eine Säule (80 cm x 5 cm) mit 200 g Kieselgel gegeben und mit Toluol äquilibriert.

Die Säule wurde mit Toluol/Essigsäureethylester (Volumenverhältnis 90:10) eluiert, wobei nach einem Vorlauf von 200 ml 500 ml einer Fraktion erhalten wurden, die eingedampft und auf einer Kieselgel-RP18-Säule (23 cm x 2,5 cm) rechromatografiert wurde. Eluiert wurde mit Methanol-Wasser (8:2). Man erhielt nach einem Vorlauf von 420 ml 100 ml einer Fraktion, welche 96 mg einer reinen Verbindung enthielt.

Diese Verbindung besitzt folgende physikalische und chemische Eigenschaften:
(1) Aussehen: Farbloses Öl
(2) Summenformel: C₂₆H₃₂O₇
(3) Molekulargewicht: 456
(4) UV-Absorptionsspektrum:
- Absorptionsmaxima bei: 230 nm (ε = 7692)
300 nm (ε = 6730)
320 nm (ε = 6730)

(5) IR-Absorptionsspektrum [cm⁻¹] (KBr-Tablette):
3450(st), 2980(st), 1710(sst), 1530(st), 1510(sst), 1435(st), 1390(w), 1290(sst), 1210(sst), 1150/1120(sst), 1040(sst), 970(st), 910(st), 810(st), 775(st).
(6) NMR-Spektrum
¹H - und ¹³C - NMR-Daten, δ-Werte)
(400 MHz bzw. 100,62 MHz MeOD als interner Standard)

| | | | |
|---|---|---|---|
| 1-H | 6.93 d 1.5 | C-1 | 115.67 |
| | | C-2 | 142.86 |
| | | C-3 | 143.46 |
| 4-H | 6.82 d 8 | C-4 | 117.74 |
| 5-H | 6.91 dd 8/1.5 | C-5 | 121.33 |
| | | C-6 | 133.56 |
| 7-H | 6.39 d 16 | C-7 | 131.24 |
| 8-H | 6.47 dd 16/10.5 | C-8 | 126.68 |
| 9-H | 6.19 d 10.5 | C-9 | 130.99 |
| | | C-10 | 131.67 |
| | | C-11 | 111.60 |
| 12-H | 7.53 s | C-12 | 160.52 |
| | | C-13 | 169.59 |
| 14-CH | 1.93 s | C-14 | 23.81 |
| 15-CH | 3.85 s | C-15 | 62.32 |
| 16-CH | 3.73 s | C-16 | 51.96 |
| 1'-H | 4.27 d 10.5 | C-1' | 66.88 |
| 1'-H | 4.07 d 10.5 | | |
| | | C-2' | 102.56 |
| | | C-3' | 84.10 |
| 4'-CH | 1.42 s | C-4' | 22.05 |
| 5'-CH | 1.32 s | C-5' | 25.14 |
| 6'-H | 5.97 d 7.5 | C-6' | 99.6 |
| 7'-H | 5.23 d 7.5 | C-7' | 123.14 |
| | | C-8' | 142.46 |
| 9'-CH | 1.73 s | C-9' | 18.31 |
| 10'-CH | 1.78 s | C-10' | 25.93 |

(7) Löslichkeit: Leicht löslich im Ethylacetat, Aceton, Ethanol und Methanol. Kaum löslich in Wasser.
(8) Dünnschichtchromatographie auf Silicagel (Kieselgel 60 F₂₅₄ E. Merck); Entwicklung mit Toluol/Ethylacetat (Volumenverhältnis 90:10): R_{f}-Wert = 0,47
(9) MS (DE 180°C):
456,21 (100, M, ber. für C₂₆H₃₂O₇ 456,27), 425(4), 372(10), 319(10), 313(10), 297(5), 256(14), 235(90), 207(14), 167(38), 153(10), 141(8), 115(8), 83(20), 75(58), 55(20), 41(22).

Gemäß Strukturanalyse besitzt die Verbindung folgende Formel:

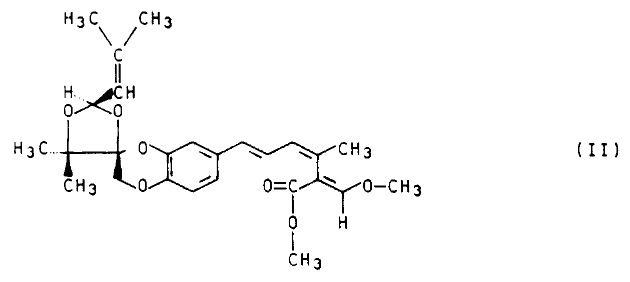

### Beispiel 2

### Atmungshemmende Wirkung bei Penicillium notatum

Der Sauerstoffverbrauch wurde in einem luftdicht abgeschlossenen Gefäß (3 ml Volumen, mit Magnetrührer) mit einer Sauerstoffelektrode polarographisch gemessen. Der Testpilz wurde aus einer Sporensuspension auf ein Mycelgewicht von 10-20 mg/ml Medium angezogen. Die Messungen wurden mit einer Mycelkonzentration von 25-30 mg Mycelfeuchtgewicht/ml in 1% iger Glucoselösung durchgeführt. Nach kurzem konstanten Atmungsverlauf wurden die in Methanol gelösten Verbindungen der Suspension zugegeben und der O₂-Verbrauch aufgezeichnet.

Der Versuch wurde unter Verwendung der Verbindung des Beispiels 1 sowie von Strobilurin A und Oudemansin A als Vergleichsverbindungen durchgeführt. Die Ergebnisse der prozentualen Hemmung der Atmung sind in Tabelle 1 genannt.

**Tabelle 1**

| Atmungshemmung in % der Kontrolle | | |
|---|---|---|
| Testverbindungen | (µg/ml) | |
| | 0,4 | 0,04 |
| Substanz des Beispiels 1 | 95 | 93 |
| Strobilurin A | 94 | 65 |
| Oudemansin A | 93 | - |

### Beispiel 3

### Wirkung auf den Einbau radioaktiver Vorstufen in Zellen von HeLa-S3 und ECA

Als Vorstufen dienten für die RNA-Biosynthese ¹⁴C-Uridin, für die DNA-Biosynthese ¹⁴C-Thymidin und für die Proteinbiosynthese ¹⁴C-Leucin.

Zu der Substanz des Beispiels 1 wurden HeLa-S3 oder ECA Zellen in einer Konzentration von 5·10⁵ bis 1·10⁶ Zellen/ml in Pbs-Puffer mit oder ohne Glucose (0,01% w/v) zugegeben. Die Zellen wurden bei 37°C auf der Schüttelmaschine 20 min mit der Substanz des Beispiels 1 vorinkubiert. Von dieser Suspension wurden je 1 ml zu den Vorstufen (0,1 µCi) gegeben und nochmals 30 min bei 37°C inkubiert. Der Einbau von radioaktiven Vorstufen wurde durch anschließende Zugabe von 1 ml eiskalter TCA (10%) gestoppt. Das säureunlösliche Präzipitat wurde auf einem Cellulosenitratfilter gesammelt und mit je 5 ml eiskalter TCA (5%) gewaschen. Nach dem Trocknen wurden die Filter mit 5 ml Scintillationsflüssigkeit überschichtet und die Radioaktivität in einem Flüssigkeitscintillationszähler bestimmt.

Die Ergebnisse sind als Prozentsatz des Gesamteinbaus bei HeLa-S3 in Tabelle 2 und bei ECA in Tabelle 3 genannt.

**Tabelle 2**

| Vorstufen | Einbau in Prozent der Kontrollen (= 100 %) Testlösung (µg/ml) | | |
|---|---|---|---|
| | 25 | 5 | 1 |
| Uridin + Glucose | 97 | 90 | 93 |
| Uridin | 64 | 60 | 64 |
| Thymidin + Glucose | 96 | 98 | 100 |
| Thymidin | 91 | 91 | 99 |
| Leucin + Glucose | 90 | 85 | 94 |
| Leucin | 90 | 88 | 89 |

**Tabelle 3**

| Vorstufen | Einbau in Prozent der Kontrollen (= 100 %) Testlösung (µg/ml) | | |
|---|---|---|---|
| | 25 | 5 | 1 |
| Uridin + Glucose | 88 | 90 | 80 |
| Uridin | 10 | 11 | 7 |
| Thymidin + Glucose | 96 | 98 | 92 |
| Thymidin | 3 | 2 | 2 |
| Leucin + Glucose | 83 | 77 | 81 |
| Leucin | 2 | 2 | 2 |

### Beispiel 4

### Cytostatische Wirkung auf HeLa-S3 Zellen

Die Inkubation von HeLa-S3 und anderen Zellen mit der erfindungsgemäßen Verbindung hemmt innerhalb kurzer Zeit die Zellteilung. Die Verbindung wirkt bei HeLa-S3 Zellen in relativ hoher Konzentration (25 µg/ml) nicht toxisch. Die Hemmung bei geringen Konzentrationen (0,025-0,25 µg/ml) kann durch Erneuern des Mediums rückgängig gemacht werden. Die Zelldichte wurde über die Bestimmung des Gesamtproteingehalts pro Loch gemessen.

Der Versuch wurde unter Verwendung der Verbindung des Beispiels 1 sowie von Strobilurin A und Oudemansin A als Vergleichsverbindungen durchgeführt. Die angeführten Werte zeigen die Ergebnisse nach dreitägiger Inkubation mit den Testsubstanzen und sind als Prozentsatz des Gesamtproteins der unbehandelten Kontrolle in Tabelle 4 aufgeführt.

**Tabelle 4**

| Testverbindung | Proteingehalt in Prozent der Kontrolle (µg/ml) | | |
|---|---|---|---|
| | 2,5 | 0,25 | 0,025 |
| Substanz des Beispiels 1 | 20 | 32 | 32 |
| Strobilurin A | 21 | 47 | 79 |
| Oudemansin A | 47 | 59 | 77 |

### Beispiel 5

### Zytotoxische und antiproliferative Wirkung auf menschliche Tumorzellen

Zur Bestimmung der antitumoralen Eigenschaften der Substanz des Beispiels 1 wurden menschliche Tumorzellen unterschiedlicher Gewebeherkunft (5637-6: Blasenkarzinom; HT-29: Kolonkarzinom; MCF-7: Mammakarzinom) verwendet.

1 bis 2·10³ sich im exponentiellen Wachstum befindliche Tumorzellen wurden in 96-Lochplatten in komplettem Wachstumsmedium (RPMI 1640 + 10% fötalem Kälberserum) ausplattiert und über Nacht bei Standardkulturbedingungen (37°C, 5% Kohlendioxid, wasserdampfgesättigte Atmosphäre) inkubiert. Die Substanzzugabe erfolgte am nächsten Tag, wobei serielle Titrationen der Substanz des Beispiels 1 über einen Konzentrationsbereich von 10⁻⁴ bis 10⁻⁹ M angelegt wurden. Nach einer weiteren Inkubation von 72 h unter Standardbedingungen erfolgte die Bestimmung der Zellzahl durch eine Färbung mit Kristallviolett und eine anschließende fotometrische Auswertung bei 540 nm mit Hilfe eines Multiphotometers.

Nur bei der sehr hohen Konzentration von 10⁻⁴ M war mikroskopisch eine Zytolyse der behandelten Zellen zu erkennen. Bei den anderen untersuchten Konzentrationen konnte eine starke, dosisabhängige Inhibition der Zellproliferation beobachtet werden, so daß auch bei der geringsten untersuchten Konzentration von 10⁻⁹ M die Zahl der behandelten Zellen einen Wert kleiner als 50% der unbehandelten Zellkontrolle zeigten.

Diese starke Abnahme der proliferativen Aktivität der mit der Substanz des Beispiels 1 behandelten Zellen geht einher mit einer morphologischen Veränderung, gekennzeichnet durch eine Streckung der Zellkörper und durch die Ausbildung von Zellfortsätzen.

### Beispiel 6

### Antivirale Wirkung bei HEp-2-Zellen auf VSV

Die Bestimmung der antiviralen Aktivität einer Testverbindung basiert auf der Messung des Schutzes von menschlichen HEp-2-Zellen als Indikatorzellen vor dem cytopathischen Effekt (CPE) von Vesicular Stomatitis Virus (VSV).

Hierzu wurden 100 µl Kulturmedium mit 2 x 10⁴ HEp-2-Zellen in die Vertiefungen einer 96-Loch-Flachbodenplatte gegeben und über Nacht bei 37°C und 5% (V/V) Kohlendioxid inkubiert. Am nächsten Tag wurden 100 µl der Probelösung zu den konfluenten Zellkulturen zugegeben und seriell 2 x titriert. Auf der Kulturplatte wurden zudem eine Zellkontrolle (= unbehandelte, nicht mit Virus infizierte Zellen) und eine Virus-kontrolle (= unbehandelte, mit Virus infizierte Zellen) mitangelegt. Nach einer weiteren Inkubationszeit von 24 h bei 37°C und 5% (V/V) CO₂ wurden die Kulturen mit 50 µl einer VSV-Suspension in Kulturmedium infiziert und bei 37°C und 5% (V/V) CO₂ inkubiert. Nach zwei Tagen war die virusbedingte Zellzerstörung (CPE) in den ungeschützten Kulturen (= Viruskontrolle) abgelaufen. Der Prozentsatz geschützter Zellen in den mit der Testverbindung behandelten und anschließend mit VSV infizierten Kulturen wurde mittels Kristallviolettfärbung bestimmt. Als Maß für die antivirale Aktivität wurde bezogen auf 0 und 100% Schutz die Probenkonzentration, die zu 50% Schutz führt, ermittelt. Als Kontrolle für die antivirale Aktivität einer Verbindung wurde rekombinantes humanes Interferon-γ (rHuIFN-γ) mituntersucht.

In Tabelle 5 ist die Testsubstanz mit der Konzentration, die die HEp-2-Zellen zu 50% vor dem zytopathischen Effekt von VSV schützt, aufgeführt.

**Tabelle 5**

| Testverbindung | Antivirale Aktivität (ng/ml) |
|---|---|
| Substanz des Beispiels 1 | 5 |
| Kontrolle (rHuIFN-γ) | 0,5 |

Im Unterschied zu rHuIFN-γ zeigte die Substanz des Beispiels 1 unter diesen Testbedingungen im Vergleich zur Zellkontrolle auch eine antiproliferative Wirkung. Die antivirale und antiproliferative Aktivitäten der Substanz des Beispiels 1 liefen parallel und korrelierten mit einer morphologischen Veränderung der Zellen, d.h. die Zellen waren im Vergleich zur Zellkontrolle langgestreckt und wiesen neuritartige Fortsätze auf.

Wurde die Substanz des Beispiels 1 bzw. rHuIFN-γ vor der VSV-Zugabe aus der Zellkultur entfernt, so blieben die Zellen vor dem CPE von VSV - wenn auch in geringerem Ausmaß - geschützt. Dies zeigt, daß die Substanz des Beispiels 1 wie für die Interferone beschrieben, nicht direkt mit den Viren reagiert, sondern die Zellen in einen Status versetzt, in dem in ihnen keine Virusvermehrung stattfinden kann.

### Beispiel 7

### Antivirale Wirkung bei BHK-21 Zellen auf VSV

Eine 96-Loch Miktrotiterplatte wurde mit BHK-21-Zellen (1·10⁴ bis 5·10⁴ Zellen/ml) angeimpft und 24 h mit G-MEM Medium (10% Serum) inkubiert. Nach dieser Zeit wurde das Medium abgehoben und die Zellen mit 25 µl VSV-Suspension infiziert (250 PFU/Loch). Nach 1 h wurden zu den Testansätzen 75 µl die Testverbindungen enthaltendes Medium (G-MEM mit 2% Serum) zugegeben. Während der Testdauer von 24 h wurden 3mal 10 µl den Zellüberständen entnommen und von diesen der Virustiter bestimmt.

Zur Titerbestimmung wurden BHK-21 Zellen auf 6-Loch Testplatten angezogen, die 10 µl-Proben wurden entsprechend verdünnt und die Zellen mit diesen Lösungen infiziert. Nach 1 h wurden die Lösungen abgehoben und die Zellen mit einem Agarmedium (1% w/v Agar, MEM-Medium ohne Phenolrot) überschichtet. Nach 24 h werden die Zellen mit Neutralrot (0,001% w/v) gefärbt und die Plaques ausgezählt.

In Tabelle 6 sind die Testverbindungen mit den eingesetzten Konzentrationen und die PFU*/Loch zur Zeit der Probenahme aufgeführt.
*PFU = Plaque forming unit

**Tabelle 6**

| Testverbindung | PFU/Loch | | |
|---|---|---|---|
| | nach 14,5 h | nach 18,5 h | nach 23,5 h |
| Substanz des Beisp. 1 (5 µg/ml) | 3,0·10² | 1,0·10³ | 1,1·10⁴ |
| Strobilurin A (5 µg/ml ) | 1,8·10² | 8,0·10² | 2,1·10³ |
| Oudemansin A (50 µg/ml) | 2,8·10² | 1,0·10³ | 9,0·10³ |
| Kontrolle | 6·1.10⁴ | 3,8·10⁵ | 1,0·10⁶ |

### Beispiel 8

### Wirkung auf die Morphologie der HeLA-S3 Zellen und die Adsorption von Pflanzenlecitin

HeLa-S3 Zellen wurden auf eine 96-Loch Titerplatte mit einer Zelldichte von 5·10⁵ bis 1·10⁶ Zellen/ml in Medium F12 aufgebracht. Nach 24 h waren die Zellen auf der Oberfläche ausgebreitet und es erfolgte ein Wechsel des Mediums. Die Zellen wurden mit dem die Testverbindungen enthaltenden Medium zweimal 72 h inkubiert. Nach 72 h besaßen die normalerweise epithelartig wachsenden Zellen eine fibroblastenartige Morphologie. Das Medium wurde anschließend durch solches ohne Testsubstanz ersetzt.

Nach 144 h wurde das Medium abgehoben, die Zellen mit PBS-Puffer gewaschen und ein mit einer Peroxidase gekoppeltes Lecitin aus Vicia villosa (spezifischer Bindung an N-Acetyl-D-galactosamin), gelöst in serumfreien F-12 Medium (75 µg/ml), zugegeben. Nach 60 min wurde das Medium abgehoben, die Zellen gewaschen und der Peroxidase Substrat-Reaktionspuffer (H₂O₂-ABTS*) zugegeben. Nach 15 min wurde die Reaktion durch Zugabe von NaN₃ gestoppt und die Absorption des oxidierten ABTS im 8-Kanal Photometer bei 405 nm gemessen.
*ABTS = 2,2' -Azino-di[3-ethyl-benzthiazolin-sulfonat]

Der Versuch wurde unter Verwendung der Verbindung des Beispiels 1 sowie von Strobilurin A und Oudemansin A als Vergleichsverbindungen durchgeführt. Die Ergebnisse sind als Prozentsatz der Gesamtaktivität von umgesetzten Substrat der unbehandelten Kontrolle in Tabelle 7 aufgeführt.

**Tabelle 7**

| Testverbindung | Lectinbindung in % der unbehandelten Kontrollen (µg/ml) | | |
|---|---|---|---|
| | 25 | 10 | 2,5 |
| Substanz des Beispiels 1 | -* | - | 44 |
| Strobilurin A | - | 67 | - |
| Oudemansin A | 67 | - | - |

| | | | |
|---|---|---|---|
| * "-" bedeutet nicht getestet | | | |

### Beispiel 9

### Antifungische Wirkung im Plattendiffusionstest

Testplatten mit Pilzen wurden einschichtig mit Medium, welches 1.5% (w/v) Agar enthält, gegossen, wobei kurz vor dem Erkalten Mycel- oder Sporensuspension zugegeben wurde. Filterrondelle (6 mm Durchmesser) wurden mit antibiotikahaltiger Lösung (10 µl) getränkt und auf die Testplatte gelegt. Das Ausmessen der Hemmhöfe erfolgte nach einer Inkubationszeit von 24 h bei 27°C. Die Ergebnisse sind in Tabelle 8 genannt. Die Konzentration der Testlösungen ist 5 µg/Rondelle.

**Tabelle 8**

| Testorganismen | Hemmhofdurchmesser (mm) |
|---|---|
| Alternaria porri | 20i* |
| Cladosporium cladosporoides | 20i |
| Curvularia lunata | 17i |
| Mucor miehei | 20 |
| Nematospora coryli | 23i |
| Neurospora crassa | 25i |
| Penicillium notatum | 15i |

| | |
|---|---|
| *i = inkompletter Hemmhof | |

### Beispiel 10

### Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 h bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.
- Bewertung:: 0 = kein Pilzbefall, abgestuft bis
5 = Totalbefall

| Wirkung | Befall der Blätter nach Applikation von 0.025%iger wäßriger Wirkstoffaufbereitung |
|---|---|
| Substanz des Beipiels 1 | 2 |
| Tridemorph | 4 |
| unbehandelt | 5 |

Die Vergleichssubstanz Tridemorph (= N-Tridecyl-2,6-dimethylmorpholin) ist als Fungizid aus DE 1 164 152 bekannt.

## Patentansprüche (Patentansprüche für folgende(n) Vertragsstaat(en): AT, BE, CH, DE, FR, GB, IT, LI, NL, SE)

1. Strobilurinderivate der Formel I

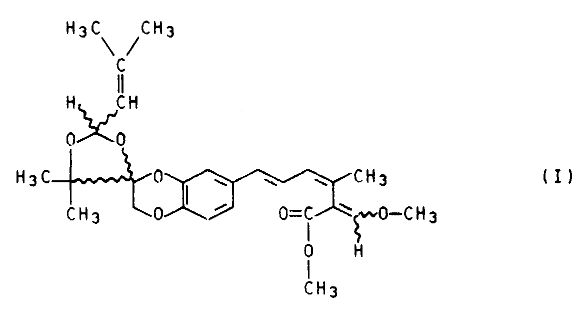

2. Verbindung der Formel

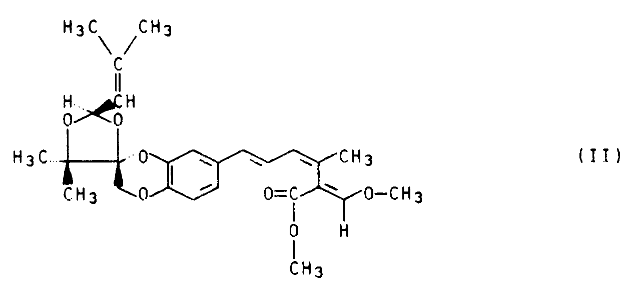

3. Verbindungen der Formel I gemäß Anspruch 1 zur Verwendung bei der Bekämpfung von Krankheiten.

4. Verwendung der Verbindungen der Formel I bei der Bekämpfung von pflanzenpathogenen Fungi.

## Patentansprüche (Patentansprüche für folgende(n) Vertragsstaat(en): ES)

1. Verfahren zur Herstellung der Strobilurinderivate der Formel I 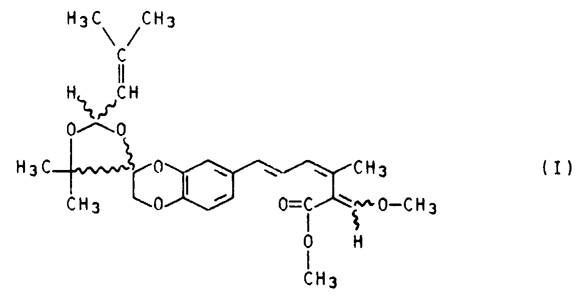 dadurch gekennzeichnet, daß man einen die Strobilurin der Formel I bildenden Mikroorganismus der Gattung Crepidotus züchtet und die Strobilurinderivate aus dem Mycel isoliert.

## Claims (Claims for the following Contracting State(s): AT, BE, CH, DE, FR, GB, IT, LI, NL, SE)

1. A strobilurine derivative of the formula I

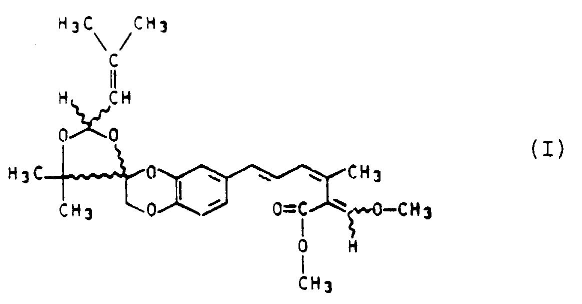

2. A compound of the formula

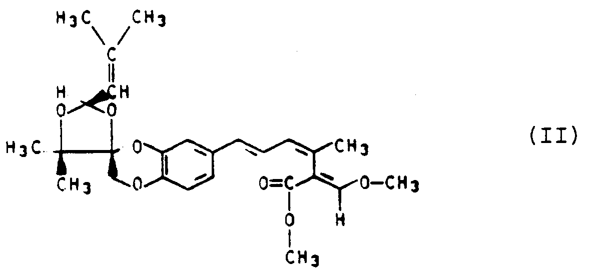

3. A compound of the formula I as claimed in claim 1 for use in the treatment of disorders.

4. Use of a compound of the formula I for controlling phytopathogenic fungi.

## Claims (Claims for the following Contracting State(s): ES)

1. A process for the preparation of the strobilurine derivative of the formula I 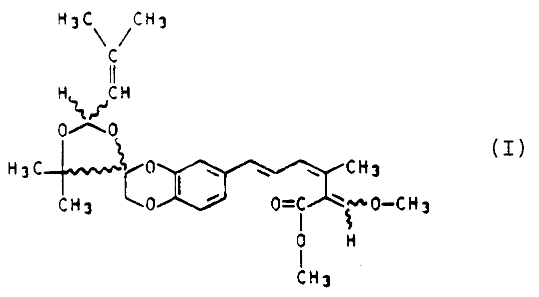 wherein a microorganism which forms the strobilurine of the formula I and is of the genus Crepidotus is cultivated and the strobilurine derivatives are isolated from the mycelium.

## Revendications (Revendications pour l'(les) Etat(s) contractant(s) suivant(s): AT, BE, CH, DE, FR, GB, IT, LI, NL, SE)

1. Dérivés de la strobilurine répondant à la formule I

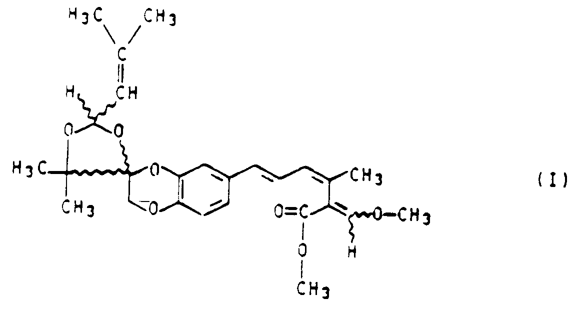

2. Composé répondant à la formule

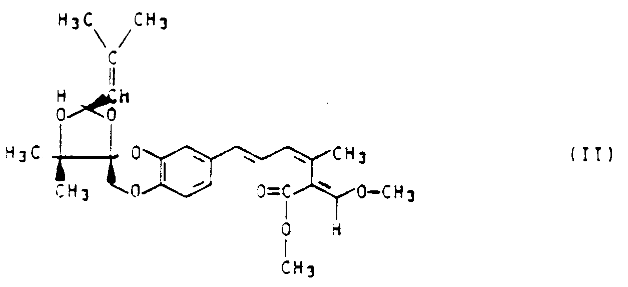

3. Composés répondant à la formule I selon la revendication 1, destinés à être utilisés pour maîtriser des maladies.

4. Utilisation des composés répondant à la formule I pour maîtriser des champignons phytopathogènes.

## Revendications (Revendications pour l'(les) Etat(s) contractant(s) suivant(s): ES)

1. Procédé pour préparer des dérivés de la strobilurine répondant à la formule I 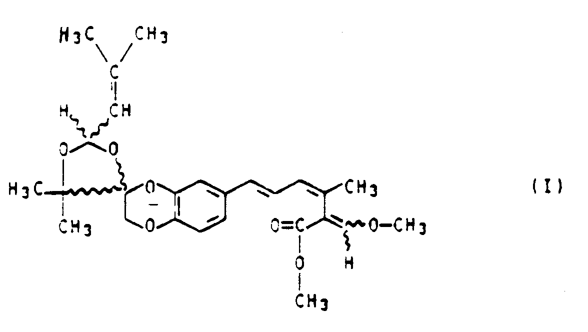 caractérisé en ce qu'on cultive un micro-organisme du genre Crepidotus, formant la strobilurine répondant à la formule I, et qu'on isole les dérivés de la strobilurine à partir du mycélium.
